# EUROPÄISCHE PATENTANMELDUNG

(11) **EP 3 441 453 A1**
(43) Veröffentlichungstag der Anmeldung: **13.02.2019**
(21) Anmeldenummer: 18188358.8
(22) Anmeldetag: 09.08.2018
(51) Int. Cl.: C12M 1/107

(54) **VERFAHREN ZUR MONTAGE EINES SENSORMODULS IN EINEM REAKTORBEHÄLTER**

(30) Priorität: 10.08.2017 DE 102017213975
(71) Anmelder: Räss, Martin, 94362 Neukirchen (DE); Schiessl, Ralf, 94362 Neukirchen (DE); Dallinger, Markus, 84329 Wurmannsquick (DE)
(72) Erfinder: Räss, Martin, 94362 Neukirchen (DE); Schiessl, Ralf, 94362 Neukirchen (DE); Dallinger, Markus, 84329 Wurmannsquick (DE)
(74) Vertreter: Benninger, Johannes

(57) **Zusammenfassung**

Es ist ein Verfahren zur Montage mindestens eines Sensors in oder an einer durch Beton oder Stahlbeton gebildeten Wandung (6) eines zumindest teilweise mit gärfähigem Material (8) oder Substrat (8) beladenen oder beladbaren Reaktorbehälters (4) offenbart. Das genannte Verfahren umfasst dabei die Verfahrensschritte: Montage eines Flansches (10) mit angesetztem Rohrstutzen (16) an einer Außenseite (6') der Wandung (6) des Reaktorbehälters (4) an der vorgesehenen Einbauposition des Sensors; Öffnen einer am Rohrstutzen (16) angeordneten und einen Durchlass (22) des Rohrstutzens (16) variabel verschließenden oder öffnenden und/oder freigebenden Verschlusseinrichtung (20); Einsetzen einer Bohrkrone in den Rohrstutzen (16) bei geöffnetem Durchlass (22) und Durchbohren der Wandung (6) bis zur Herstellung einer in einen Innenraum (4') des Reaktorbehälters (4) reichenden Durchgangsbohrung; Herausziehen der Bohrkrone aus dem Rohrstutzen (16) und unmittelbar darauf folgendes Verschließen der Verschlusseinrichtung (20); Öffnen der Verschlusseinrichtung (20) und unmittelbar darauf folgendes Montieren eines Sensormoduls, dessen Modulschaft weitgehend dichtend im Durchlass (22) des Rohrstutzens (16) positioniert wird, wobei mindestens ein mit einem zur Erfassung wenigstens eines Parameters des im Reaktorbehälter (4) befindlichen Gärsubstrats (8) vorgesehener und/oder ausgebildeter Sensor an einem in den Innenraum (4') des Reaktorbehälters (4) ragenden Abschnitt des Modulschafts angeordnet ist.

## Beschreibung

Die vorliegende Erfindung betrifft ein Verfahren zur Montage eines Sensormoduls in einem zumindest teilweise mit gärfähigem Material beladenen oder beladbaren Reaktorbehälter mit den Merkmalen des unabhängigen Verfahrensanspruchs 1.

Die Gärprozesse innerhalb der Gärreaktorbehälter von Biogasanlagen sind für die unterschiedlichsten zu vergärenden Substrate in groben Zügen bekannt und zumindest teilweise von außen steuerbar, so etwa mittels Rührwerken, durch Zugabe von die Gärung unterstützenden Substanzen, durch Zugabe von gärfähigem Substrat, durch Zugabe oder Entnahme von Flüssigkeit, durch Entnahme von durch die Gärung entstandenem Gas, durch Variation und/oder Steuerung der Temperaturen im Gärbehälter etc.

Dennoch ist es zur besseren Überwachung der im Gärreaktor stattfindenden Prozesse in vielen Fällen wünschenswert, mittels unterhalb des Flüssigkeitsspiegels des Gärsubstrats angeordneter Sensoren die Vorgänge innerhalb des Gärsubstrats genauer überwachen und/oder den Zustand des Substrats beurteilen zu können, was insbesondere mittels im Reaktorbehälter angeordneter Sensorsysteme möglich ist. Mittels des Einsatzes solcher Sensorsysteme sind nicht nur Fernüberwachungen in Echtzeit ermöglicht, sondern derartige Sensorsysteme können es je nach Signalverarbeitung darüber hinaus ermöglichen, eine Aufzeichnung und Speicherung der erfassten Sensorwerte vorzunehmen, um etwa eine ständige oder in zyklischen Abständen erfolgende Analyse der Gärprozesse durchzuführen.

Üblicherweise wird die Montage derartiger Sensorsysteme bei leerem Reaktorbehälter durchgeführt. Die Montage ist hierbei recht einfach durchführbar, denn bei leerem Reaktorbehälter sind die Bestimmungsorte der Einrichtungen gut zugänglich, da sich kein gärfähiges Material oder Substrat im Reaktorbehälter befindet und der Reaktorbehälter bei Bedarf ggf. auch betreten werden kann. Für die Montage eines Sensorsystems im oder am Reaktorbehälter bietet sich also insbesondere Zeiträume an, in denen der Reaktorbehälter unbeladen ist. Derartige Zeiträume stehen beispielsweise direkt nach Fertigstellung des Reaktorbehälters bzw. der Biogasanlage zur Verfügung, bevor diese in Betrieb genommen und der Reaktorbehälter mit gärfähigem Material oder Substrat beladen wird. Auch bei möglicherweise notwendigen Wartungsarbeiten im Innenraum des Reaktorbehälters oder am Reaktorbehälter kann der Reaktorbehälter für die Anbringung eines Sensorsystems zugänglich sein. Alternativ können auch Zeiträume zur Verfügung stehen, in denen eine Entleerung durchgeführt wurde, um den Reaktorbehälter anschließend neu zu befüllen. Erfahrungsgemäß stehen derartige Zeiträume mit zugänglichem Reaktorbehälter jedoch selten und nur in großen zeitlichen Abständen zur Verfügung.

Um ein Sensorsystem im oder am Reaktorbehälter anzubringen, kann der Reaktorbehälter zudem auch im laufenden Betrieb entleert werden, um das Sensorsystem im oder am Reaktorbehälter zu installieren. Eine eigens für die Montage eines Sensorsystems durchzuführende Entleerung des Reaktorbehälters und die somit stattfindende Unterbrechung des Gärprozesses führen jedoch zu hohen zusätzlichen und insbesondere unplanmäßigen Betriebskosten sowie zu Störungen im Betriebsplan.

Angesichts der im Stand der Technik identifizierten Nachteile kann es als vorrangiges Ziel der vorliegenden Erfindung betrachtet werden, ein Verfahren zur Montage eines Sensors in oder an einer Wandung eines Reaktorbehälters zur Verfügung zu stellen, ohne dabei einen Zeitraum abwarten zu müssen, in welchem der Reaktorbehälter frei zugänglich ist, oder ohne den Reaktorbehälter entleeren zu müssen. Zudem soll die Montage eines Sensors einfach sowie kostengünstig durchzuführen sein.

Diese identifizierten Ziele werden mit dem Gegenstand des unabhängigen Verfahrensanspruchs 1 erreicht. Merkmale vorteilhafter Weiterbildungen der Erfindung finden sich in den abhängigen Ansprüchen.

Zur Erreichung des genannten Ziels schlägt die Erfindung ein Verfahren zur Montage mindestens eines Sensors in oder an einer durch Beton oder Stahlbeton gebildeten Wandung eines zumindest teilweise mit gärfähigem Material oder Substrat oder ggf. auch mit einer Flüssigkeit beladenen oder beladbaren Reaktorbehälters vor. Der Reaktorbehälter kann hierbei beispielsweise Teil einer Biogasanlage sein. Das genannte erfindungsgemäße Verfahren zur Montage mindestens eines Sensors in oder an einer Wandung eines zumindest teilweise mit gärfähigem Material oder Substrat, kurz Gärsubstrat, beladenen oder beladbaren Reaktorbehälters umfasst dabei zumindest folgende Verfahrensschritte:
- Montage eines Flansches mit angesetztem Rohrstutzen an einer Außenseite der Wandung des Reaktorbehälters an der vorgesehenen Einbauposition des Sensors,
- Öffnen einer am Rohrstutzen angeordneten und einen Durchlass des Rohrstutzens variabel verschließenden oder öffnenden und/oder freigebenden Verschlusseinrichtung,
- Einsetzen einer Bohrkrone in den Rohrstutzen bei geöffnetem Durchlass und Durchbohren der Wandung bis zur Herstellung einer in einen Innenraum des Reaktorbehälters reichenden Durchgangsbohrung,
- Herausziehen der Bohrkrone aus dem Rohrstutzen und unmittelbar darauf folgendes Verschließen der Verschlusseinrichtung,
- Öffnen der Verschlusseinrichtung und unmittelbar darauf folgendes Montieren eines Sensormoduls, dessen Modulschaft weitgehend dichtend im Durchlass des Rohrstutzens positioniert wird, wobei mindestens ein mit einem zur Erfassung wenigstens eines Parameters des im Reaktorbehälter befindlichen Gärsubstrats vorgesehener und/oder ausgebildeter Sensor an einem in den Innenraum des Reaktorbehälters ragenden Abschnitt des Modulschafts angeordnet ist.

Gemäß dem erfindungsgemäßen Verfahren kann also mindestens ein Sensor in oder an der Wandung des gefüllten Reaktorbehälters montiert werden. In einem ersten Schritt soll dabei ein Flansch mit einem angesetzten Rohrstutzen an einer Außenseite der Wandung des Reaktorbehälters angelegt und anschließend an einer vorgesehenen Einbauposition angebracht werden.

In einer bevorzugten Ausführungsform des erfindungsgemäßen Verfahrens wird der Flansch in einem Bereich der Wandung montiert, welche bei mit Gärsubstrat beladenem Reaktorbehälter unterhalb einer Gasphase und/oder unterhalb eines Flüssigkeitsspiegels angeordnet ist. Üblicherweise sind derartige Reaktorbehälter mit einer Folienhaube versehen, unterhalb welcher sich bei einer Gärreaktion des Gärsubstrats entstehendes oder entstandenes Gas sammelt. Bei dem genannten Gas kann es sich insbesondere um Methangas (CH₄) handeln, welches ggf. bei Kontakt mit Luft ein explosives Gemisch bilden kann. Zum Schutz vor einer möglichen Explosion aufgrund eines entzündbaren Methan-Luft-Gemisches unterliegen die Reaktorbehälter üblicherweise strengen Explosionsschutzauflagen. Eine bevorzugte Einbauposition des mindestens einen Sensors befindet sich daher vorzugsweise in einem gewissen Abstand unterhalb der Gasphase bzw. unterhalb des Flüssigkeitsspiegels des Gärsubstrats, um außerhalb der Explosionszone des Gärgases zu bleiben. Beispielsweise kann sich so eine Einbauposition für den mindestens einen Sensor, bzw. auch des Flansches mit angesetztem Rohrstutzen ergeben, welche rund 2-3m, beispielsweise ca. 2,5m, unterhalb des Gasspiegels liegt. Dadurch kann die Explosionsgefahr bei der Montage oder auch beim Betrieb des mindestens einen Sensors erheblich minimiert oder sogar eliminiert werden. Zudem können bei derartiger Anordnung herkömmliche Sensoren, Flansche oder sonstige Bauteile verwendet werden, welche nicht den Explosionsschutzrichtlinien entsprechen müssen. Derartige herkömmliche Bauteile sind in der Regel erheblich kostengünstiger in der Anschaffung als Bauteile nach den angesprochenen Richtlinien.

Der Flansch kann hierbei mit geeigneten Befestigungsmitteln (dauerhaft oder mittels Werkzeugen lösbar) an der Außenseite der Wandung befestigt werden. Eine an der Wandung des Reaktorbehälters anliegende Flanschfläche des Flansches kann eine mit einer Außenkontur und/oder Wandkrümmung der Wandung des Reaktorbehälters korrespondierende Kontur aufweisen. Vorzugsweise ist dabei der Rohrstutzen bei montiertem Flansch ungefähr rechtwinkelig zu einer Oberfläche der Wandung des Reaktorbehälters orientiert. Wie bereits erwähnt, ist der Rohrstutzen mit einer Verschlusseinrichtung versehen, um den Durchlass am Rohrstutzen variabel verschließen oder öffnen und/oder freigeben zu können. Beispielsweise kann die Verschlusseinrichtung als Absperrschieber ausgebildet sein.

Weiterhin kann der Durchlass am Rohrstutzen langsam geöffnet werden, um schließlich eine Bohrkrone bei ausreichend geöffnetem Durchlass in den Rohrstutzen einsetzen zu können. Die Bohrkrone kann so bis zur Wandung durch den Rohrstutzen vordringen bzw. die Wandung durchbohren, um eine in den Innenraum des Reaktorbehälters reichende Durchgangsbohrung herzustellen. Anschließend kann die Bohrkrone wieder aus dem Rohrstutzen herausgezogen werden. Um das Herausfließen oder Austreten von Gärsubstrat aus dem Reaktorbehälter über den Rohrstutzen zu verhindern, kann ein unmittelbar auf das Herausziehen der Bohrkrone aus dem Rohrstutzen vorgesehenes Verschließen der Verschlusseinrichtung notwendig sein.

Zur Montage des Sensormoduls in bzw. an der Wandung des Reaktorbehälters kann die Verschlusseinrichtung wiederum geöffnet werden. Das Sensormodul kann hierbei mit mindestens einem geeigneten Sensor versehen sein. Der Modulschaft des Sensormoduls kann dabei weitgehend abdichtend im Durchlass des Rohrstutzens positioniert werden, um das Austreten von Gärsubstrat zu vermeiden. Durch den Rohrstutzen kann das Sensormodul bzw. der mindestens eine Sensor in dessen endgültigen Bestimmungsort gebracht werden. Der mindestens eine Sensor kann dabei derartig an dem Modulschaft des Sensormoduls angeordnet sein, sodass der mindestens eine Sensor nach Abschluss der Montage des Sensormoduls in den Innenraum des Reaktorbehälters ragt.

Wie bereits erwähnt kann der mindestens eine Sensor zur Erfassung wenigstens eines Parameters des im Reaktorbehälter befindlichen gärfähigem Material oder Substrat vorgesehen und/oder ausgebildet sein. Für das Sensormodul können unterschiedliche Sensoren genutzt werden. Hierbei kann der wenigstens eine Sensor des Sensormoduls beispielsweise durch einen Kraftsensor, hierbei insbesondere in Form einer Wägezelle, durch einen Temperatursensor, durch einen Strömungssensor oder auch durch eine optische Erfassungseinrichtung gebildet sein oder eine derartigen Sensor bzw. eine derartige Einrichtung umfassen.

Weiterhin kann vorgesehen sein, dass ein nach außen aus dem Rohrstutzen reichender Abschnitt des Modulschafts mit elektrischen Anschlüssen und/oder elektrischen Leitungen zur Übermittlung von elektrischen Sensorsignalen des mindestens einen Sensors des Sensormoduls ausgestattet ist. Zudem kann der mindestens eine Sensor die Zustandsparameter des im Erfassungsbereich des Sensormoduls befindlichen Innenraumes des Reaktorbehälters, insbesondere seiner Materialfüllung und/oder der darin befindlichen Flüssigkeit in elektrische Signale wandeln, die mittels Datenverarbeitungseinrichtungen ausgewertet und/oder verarbeitet werden.

Sofern geeignete Einrichtungen vorhanden sind, können die so ermittelten Daten auch an einen sogenannten Cloud-Speicher übertragen werden. Auf diese Weise ist es nicht notwendig, lange Datenleitungen ausgehend von der Datenverarbeitungseinrichtung zu verlegen, sondern es kann zur Datenübertragung auch eine Internetanbindung genutzt werden. So ist es nicht unbedingt nötig, einen Überwachungsposten vor Ort ständig zu besetzen, sondern Bedienpersonen können über den Cloud-Speicher von unterschiedlichen Orten aus auf die ermittelten Daten zugreifen.

Weiterhin betrifft die vorliegende Erfindung einen Reaktorbehälter, der eine durch Beton oder Stahlbeton gebildete Wandung aufweist, und der zumindest teilweise mit gärfähigem Material oder Substrat beladen oder beladbar ist. Zudem weist der Reaktorbehälter mindestens einen Flansch mit darin montiertem Sensormodul auf, wobei der Flansch gemäß einer Ausführungsform des zuvor beschriebenen Verfahrens montiert wurde. Auch zählt eine Biogasanlage zur vorliegenden Erfindung, welche einen derartigen Reaktorbehälter aufweist, welcher mit mindestens einem Sensormodul ausgestattet ist, welches gemäß einer Ausführungsform des zuvor beschriebenen Verfahrens montiert wurde.

Es sei an dieser Stelle ausdrücklich klargestellt, dass alle Aspekte, die im Zusammenhang mit dem erfindungsgemäßen Verfahren beschrieben wurden, gleichermaßen für den Reaktorbehälter gelten können, der mit einem Sensormodul nach vorliegendem erfindungsgemäßen Verfahren ausgestattet wurde. Gleiches gilt für die mit einem solchen Reaktorbehälter oder mit mehreren solchen Reaktorbehältern ausgestattete Biogasanlage. Alle genannten Aspekte und Merkmale können daher gleichermaßen auf einen Reaktorbehälter und/oder eine Biogasanlage mit mindestens einem Reaktorbehälter gelesen und verstanden werden.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.
Fig. 1 zeigt einen ersten Montageschritt zur Montage eines Sensors an einem Reaktorbehälter.
Fig. 2 zeigt einen weiteren Montageschritt zur Montage eines Sensors an einem Reaktorbehälter.
Fig. 3 zeigt einen weiteren, folgenden Montageschritt zur Montage eines Sensors an einem Reaktorbehälter.
Fig. 4 zeigt einen weiteren, folgenden Montageschritt zur Montage eines an einem Reaktorbehälter.
Fig. 5 zeigt einen weiteren, folgenden Montageschritt zur Montage eines Sensors an einem Reaktorbehälter.
Fig. 6 zeigt einen weiteren, folgenden Montageschritt zur Montage eines Sensors an einem Reaktorbehälter.
Fig. 7 zeigt einen erfindungsgemäß montierten Sensor in bzw. an einer Wandung eines Reaktorbehälters.
Fig. 8 zeigt eine weitere Ausführungsform zweier in bzw. an einer Wandung eines Reaktorbehälters montierter Sensormodule.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellte Ausführungsform stellt lediglich ein Beispiel dar, wie das erfindungsgemäße Verfahren durchgeführt werden kann, soll jedoch nicht als abschließende Begrenzung aufgefasst werden.

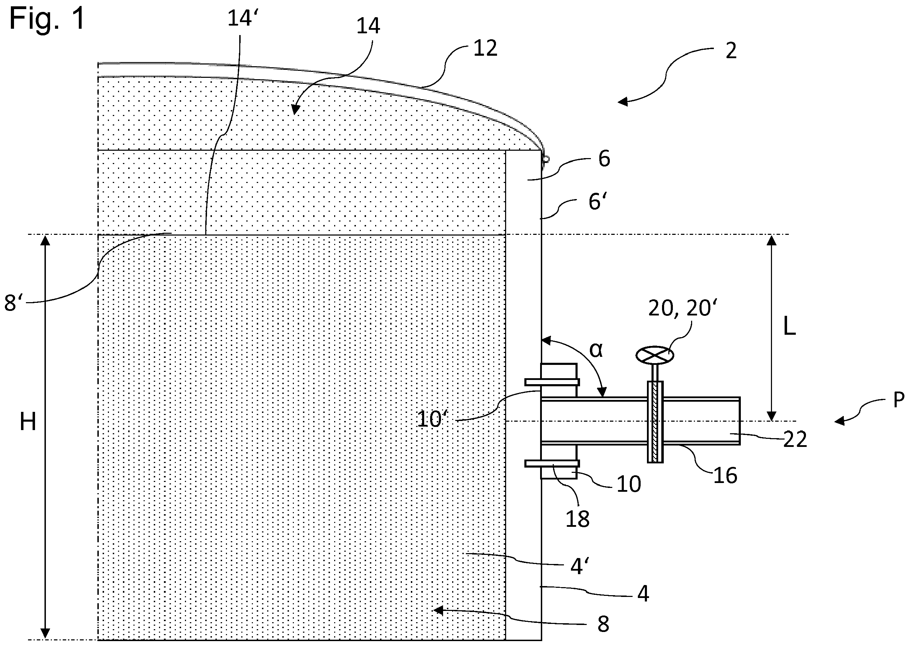

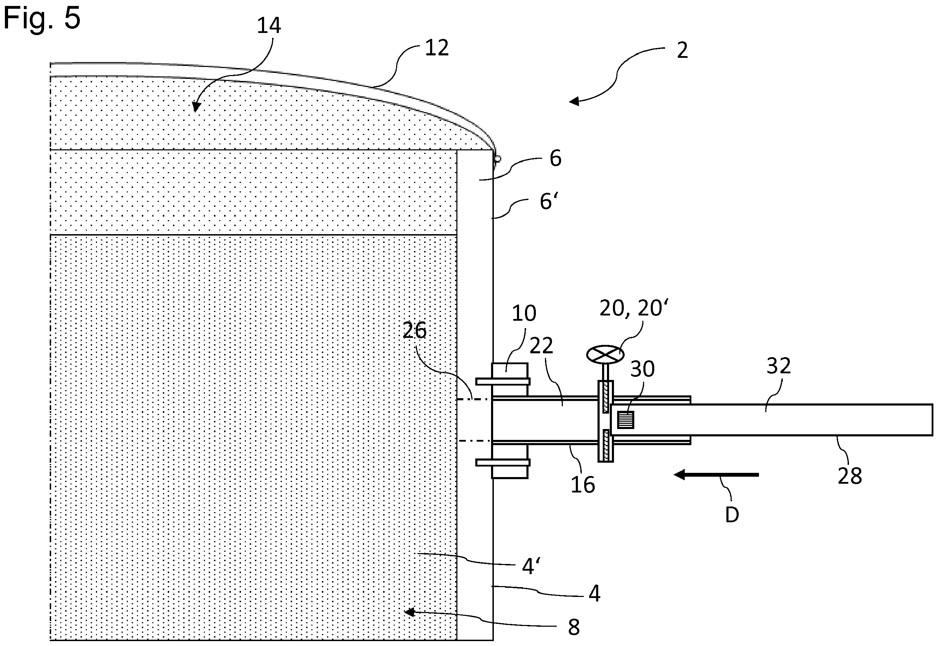

Die schematische Teilschnittdarstellung der Fig. 1 zeigt ein Anwendungsbeispiel eines erfindungsgemäßen Verfahrens zur Montage mindestens eines Sensors 30 (vgl. z.B. Fig. 5-7) in oder an einer Wandung 6 eines Reaktorbehälters 4. Der Reaktorbehälter 4 ist hierbei vorzugsweise Teil einer Biogasanlage 2. Die Wandung 6 des Reaktorbehälters 4 kann hierbei beispielsweise durch Beton oder Stahlbeton ausgebildet sein. Bei Durchführung des erfindungsgemäßen Verfahrens kann der Reaktorbehälter 4 insbesondere zumindest teilweise mit gärfähigem Material 8 oder Substrat 8, kurz Gärsubstrat 8, gefüllt bzw. beladen sein. In der Abbildung der Fig. 1 ist der Reaktorbehälter 4 beispielhaft bis zur Höhe H mit Gärsubstrat 8 beladen. Weiterhin ist der Reaktorbehälter 4 mit einer Folienhaube 12 versehen. Unterhalb der Folienhaube 12 wird bei der Gärreaktion des Gärsubstrats 8 entstehendes oder entstandenes Gas 14, beispielsweise Methangas (CH₄), gespeichert.

Gemäß dem vorliegenden Verfahren soll nun ein Sensor 30 in der durch, wie bereits erwähnt, beispielsweise durch Beton oder Stahlbeton ausgebildeten Wandung 6 des mit Gärsubstrat 8 gefüllten Reaktorbehälters 4 montiert werden. In einem ersten Schritt wird ein Flansch 10 mit einem angesetzten Rohrstutzen 16 an einer Außenseite 6' der Wandung 6 des Reaktorbehälters 4 angelegt und anschließend in einer vorgesehenen Einbauposition montiert. Die vorgesehene Einbauposition des Sensors 30 befindet sich in einem mit L gekennzeichneten Abstand unterhalb des Gasspiegels 14'. Bei der Wahl der Einbauposition P ist es wichtig, eine Position unterhalb des Gasspiegels 14' bzw. unterhalb des Flüssigkeitsspiegels 8' des Gärsubstrats 8 zu wählen, um außerhalb der Explosionszone des Gases 14 zu bleiben. Hierdurch kann die Explosionsgefahr bei der Montage oder auch beim Betrieb des Sensors 30 minimiert oder sogar eliminiert werden. Es können somit herkömmliche Sensoren 30, Flansche 10 oder sonstige Bauteile verwendet werden, welche nicht den Explosionsschutzrichtlinien entsprechen müssen. Beispielsweise kann sich so eine Einbauposition P für den Sensor 30, bzw. auch des Flansches 10 mit angesetztem Rohrstutzen 16 ergeben, welche rund 2,5m unterhalb des Gasspiegels 14' liegt.

Der Flansch 10 kann hierbei mit geeigneten Befestigungsmitteln 18 (dauerhaft) an der Außenseite 6' der Wandung 6 befestigt werden. Hierbei kann die an der Wandung 6 anliegende Flanschfläche 10' des Flansches 10 eine mit einer Außenkontur bzw. Wandkrümmung der Wandung 6 des Reaktorbehälters 4 korrespondierende Kontur aufweisen. Vorzugsweise wird der Flansch 10 derartig an der Wandung 6 befestigt, sodass sich zwischen der Oberfläche der Wandung 6 und dem Rohrstutzen 16 ein Winkel α von vorzugsweise 90° ergibt. Natürlich ist auch die Ausbildung anderer Beträge des Winkels α zwischen der Oberfläche der Wandung 6 und dem Rohrstutzen 16 denkbar. Der Rohrstutzen 16 ist mit einer Verschlusseinrichtung 20 versehen, um einen Durchlass 22 am Rohrstutzen 16 variabel verschließen oder öffnen und/oder freigeben zu können. Beispielsweise kann die Verschlusseinrichtung 20 als Absperrschieber 20' ausgebildet sein.

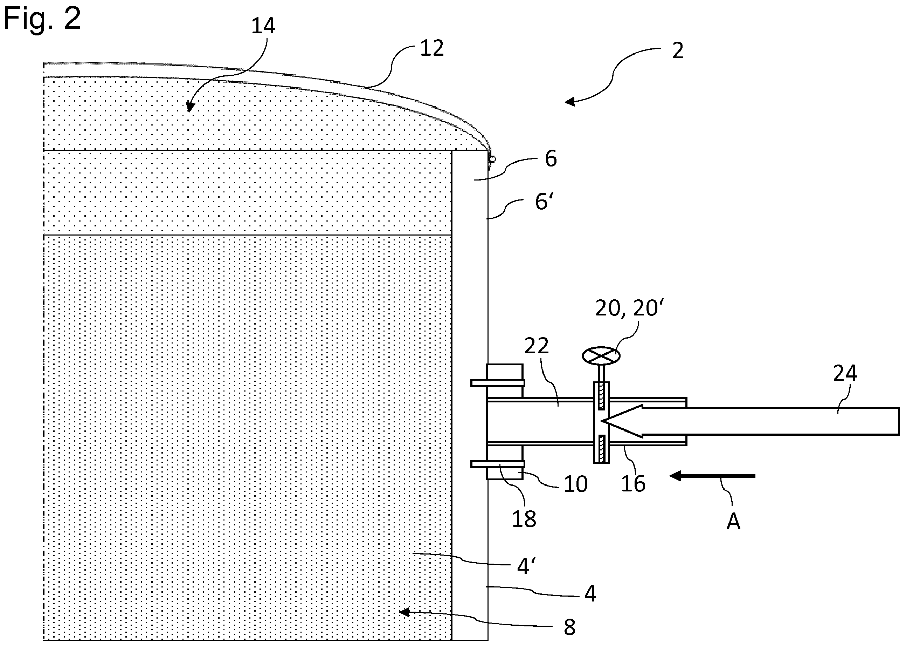

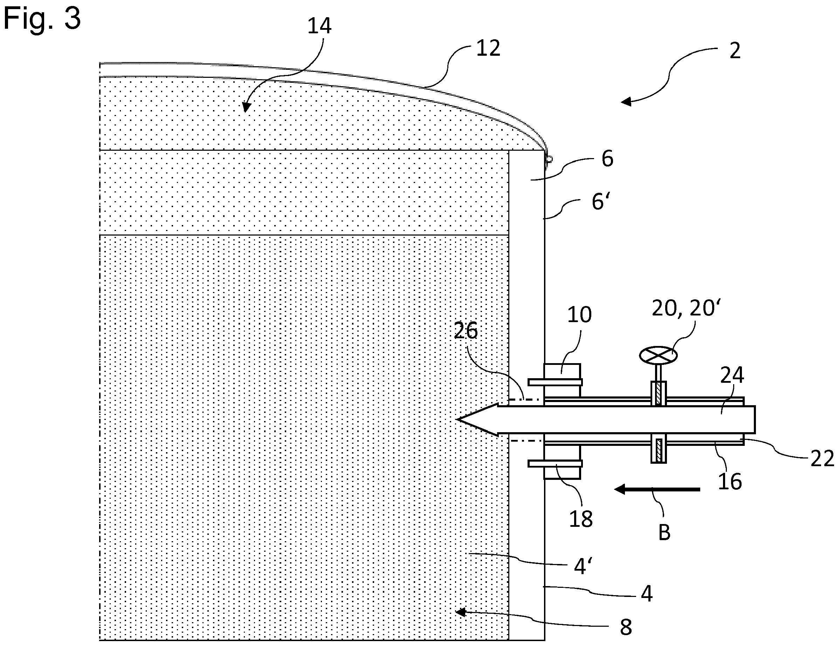

Im folgenden Teilschritt des erfindungsgemäßen Verfahrens, welcher durch die Fig. 2 angedeutet ist, wird nun der Durchlass 22 über die Verschlusseinrichtung 20 am Rohrstutzen 16 langsam geöffnet. Anschließend kann eine Bohrkrone 24 bei ausreichend geöffnetem Durchlass 22 bzw. bei ausreichend geöffneter Verschlusseinrichtung 20 in den Rohrstutzen 16 eingesetzt werden, was durch den Pfeil A symbolisiert wird. In der folgenden Fig. 3 ist die Bohrkrone 24 bis zur Wandung 4 durch den Rohrstutzen 16 vorgedrungen bzw. hat bereits die Wandung 6 durchbohrt, um eine in den Innenraum 4' des Reaktorbehälters 4 reichende Durchgangsbohrung 26 herzustellen. Dieser Vorgang ist durch den Pfeil B angedeutet.

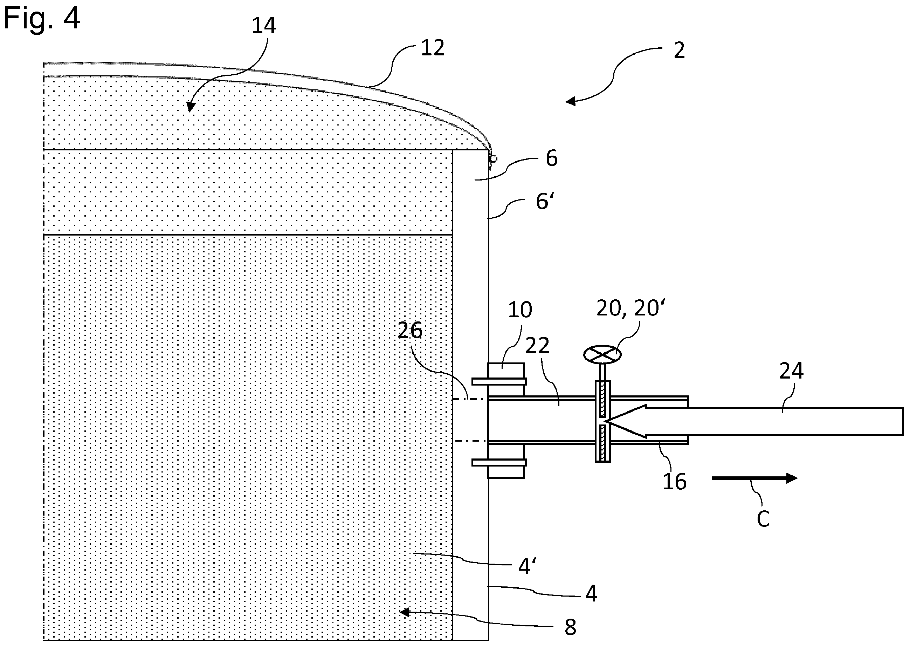

Die schematische Ansicht der Fig. 4 zeigt einen weiteren Verfahrensschritt des erfindungsgemäßen Verfahrens. Hier wird die Bohrkrone 24 bereits wieder aus dem Rohrstutzen 16 herausgezogen (vgl. Pfeil C). Um das Herausfließen oder Austreten von Gärsubstrat 8 aus dem Reaktorbehälter 4 über den Rohrstutzen 16 zu verhindern, ist ein unmittelbar auf das Herausziehen der Bohrkrone 24 aus dem Rohrstutzen 16 vorgesehenes Verschließen der Verschlusseinrichtung 20 vorgesehen.

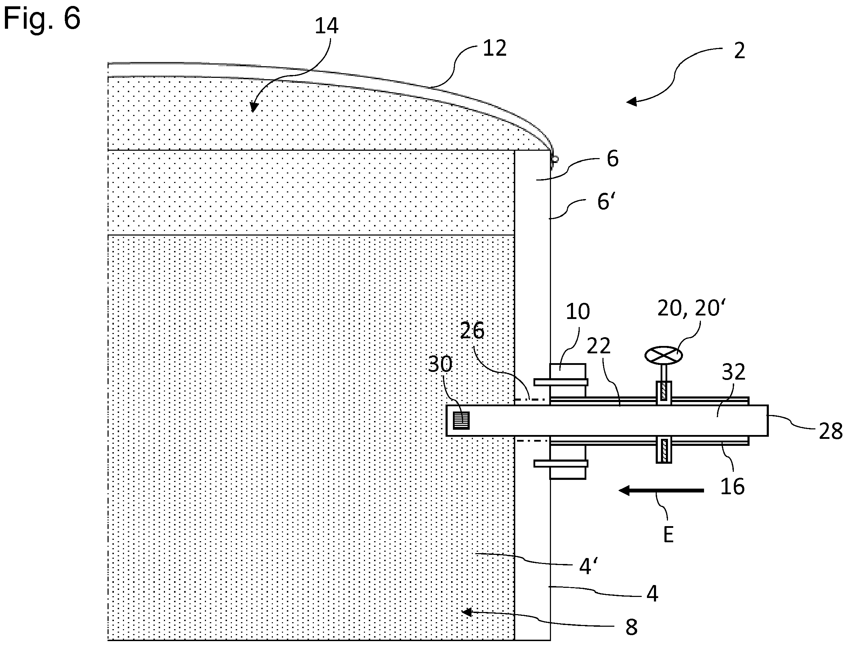

Im der folgenden Fig. 5 ist nun gezeigt, wie ein Sensormodul 28 montiert wird. Hierzu wird die Verschlusseinrichtung 20 wiederum geöffnet. Unmittelbar darauf folgt das Montieren des Sensormoduls 28. Das Sensormodul 28 ist hierbei mit einem geeigneten Sensor 30 versehen. Der Modulschaft 32 des Sensormoduls 28 wird dabei weitgehend abdichtend im Durchlass 22 des Rohrstutzens 16 positioniert, wie der Pfeil D andeutet. In Fig. 6 ist das den Sensor 30 aufweisende Sensormodul 28 bereits in seine endgültige Bestimmungsposition durch den Rohrstutzen 16 verbracht (vgl. Pfeil E). Der Sensor 30 ist dabei derartig an dem Modulschaft 32 des Sensormoduls 28 angeordnet, sodass der Sensor 30 nach Abschluss der Montage des Sensormoduls 28 in den Innenraum 4' des Reaktorbehälters 4 ragt. Der Sensor 30 kann zur Erfassung wenigstens eines Parameters des im Reaktorbehälter 4 befindlichen Gärsubstrats 8 vorgesehen und/oder ausgebildet sein.

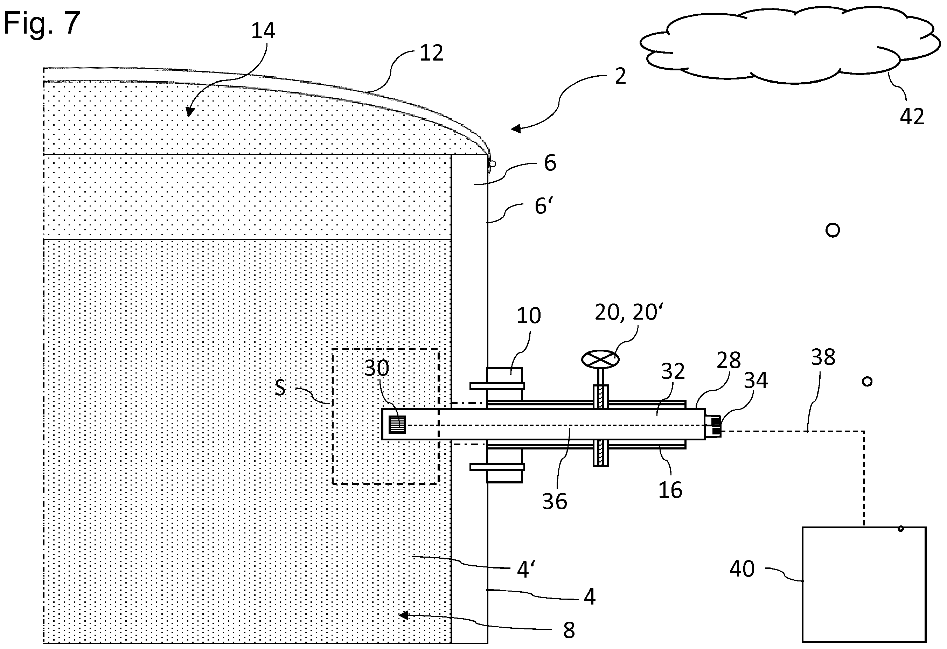

Die schematische Ansicht der Fig. 7 zeigt ein anhand des erfindungsgemäßen Verfahrens montiertes Sensormodul 28. Ein nach außen aus dem Rohrstutzen reichender Abschnitt des Modulschafts 32 des Sensormoduls 28 ist dabei mit elektrischen Anschlüssen 34 und/oder elektrischen Leitungen 36 zur Übermittlung von elektrischen Sensorsignalen 38 des wenigstens einen Sensors 30 des Sensormoduls 28 ausgestattet. Bei dem Sensor 30, welcher für das Sensormodul 28 genutzt wird, kann es sich um unterschiedliche Sensoreinrichtungen handeln. So ist es denkbar, dass der Sensor des Sensormoduls durch einen Kraftsensor, insbesondere in Form einer Wägezelle, durch einen Temperatursensor, durch einen Strömungssensor oder durch eine optische Erfassungseinrichtung ausgebildet ist oder einen der genannten Einrichtungen umfasst. Ebenso kann es auch denkbar sein, mehrere der genannten Sensoren in einem Sensormodul zu verwenden. Der Sensor 30 kann die Zustandsparameter des beispielhaft im mit S gekennzeichneten Erfassungsbereich des Sensormoduls 28 befindlichen Innenraums 4' des Reaktorbehälters 4 in elektrische Signale 38 umwandeln. Hierbei können die Zustandsparameter insbesondere einen Zustand der sich im Reaktorbehälter 4 befindenden Material- 8 oder Substratfüllung 8 und/oder einer sich im Innenraum 4' des Reaktorbehälters 4 befindenden Flüssigkeit betreffen, welche anschließend in weiterverwertbare elektrische Signale 38 umgewandelt werden können. Mittels geeigneter Datenverarbeitungseinrichtungen 40 können die so erhaltenen Zustandsparameter bzw. die elektrischen Signale 38 ausgewertet und/oder verarbeitet werden.

Sofern geeignete Einrichtungen vorhanden sind, können die ermittelten Daten an einen Cloud-Speicher 42 übertragen werden. Auf diese Weise ist es nicht notwendig, lange Datenleitungen ausgehend von der Datenverarbeitungseinrichtung 40 zu verlegen, sondern es kann zur Datenübertragung auch das Internet verwendet werden. So ist es zudem nicht nötig, ständig einen Überwachungsposten vor Ort zu besetzen, sondern Bedienpersonen können über den Cloud-Speicher 42 von unterschiedlichen Orten aus auf die Daten zugreifen.

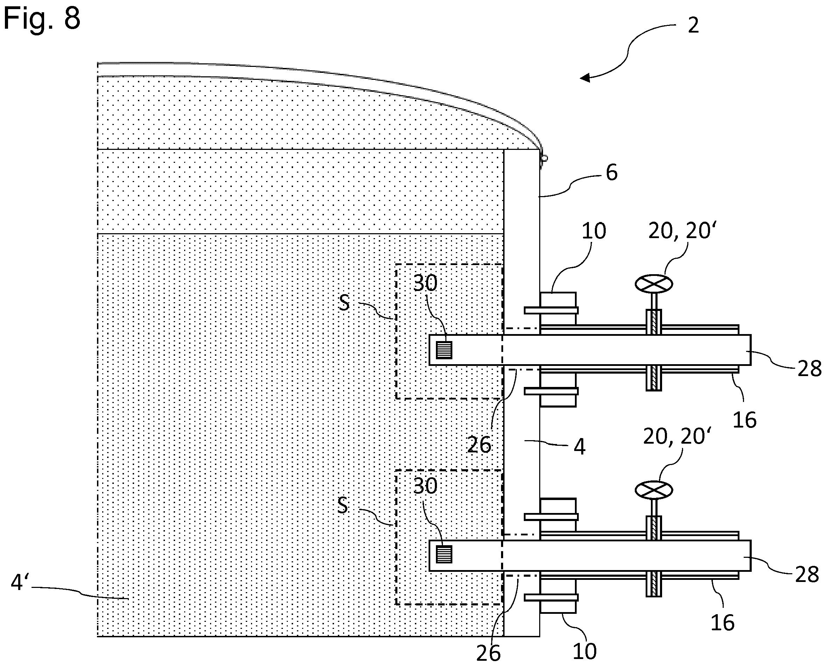

Die schematische Ansicht der Fig. 8 zeigt eine weitere Ausführungsform der vorliegenden Erfindung. Hierbei sind zwei mit einem Rohrstutzen 16 versehene Flansche 10 an der Wandung 6 eines Reaktorbehälters 4 angeordnet. Anhand des zuvor beschriebenen Verfahrens wurden mittels Bohrkronen 24 bereits Durchgangsbohrungen 26 durch die Wandung 6 des Reaktorbehälters 4 erstellt, um vorzugsweise ebenfalls zuvor beschriebene Sensormodule 28 in den Innenraum 4' des Reaktorbehälters 4 zu verbringen. Hierbei ist es durchaus denkbar, gleiche oder unterschiedliche Sensoren 30 an den Sensormodulen 28 zu nutzen.

Wie schon oben an anderer Stelle erwähnt, kann der Reaktorbehälter 4 mit dem ihm zugeordneten Sensormodul 28 insbesondere ein Bestandteil einer komplexeren Biogasanlage 2 sein, in der aus gärfähigen Substraten 8 oder Materialien 8 durch Gären und weitere Umwandlungsprozesse brennbare und als Brennstoff für Motoren oder Feuerungsanlagen verwendbare Gase wie etwa Methangas gebildet werden.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

- 2: Biogasanlage
- 4: Reaktorbehälter
- 4': Innenraum des Reaktorbehälters
- 6: Wandung
- 6': Außenseite der Wandung
- 8: Material, Substrat, Gärsubstrat
- 8': Flüssigkeitsspiegel
- 10: Flansch
- 10': Flanschfläche
- 12: Folienhaube
- 14: Gas
- 14': Gasspiegel, Gasphase
- 16: Rohrstutzen
- 18: Befestigungsmittel
- 20: Verschlusseinrichtung
- 20': Absperrschieber
- 22: Durchlass
- 24: Bohrkrone
- 26: Durchgangsbohrung
- 28: Sensormodul
- 30: Sensor
- 32: Modulschaft
- 34: elektrische Anschlüsse
- 36: elektrische Leitungen
- 38: elektrische Sensorsignale
- 40: Datenverarbeitungseinrichtung
- 42: Cloud-Speicher

- L: Abstand des Flansches zum Gasspiegel
- H: Füllhöhe des Gärsubstrats im Reaktorbehälter
- P: Einbauposition des Flansches
- S: Erfassungsbereich des Sensors bzw. des Sensormoduls
- α: Winkel zwischen der Oberfläche der Wandung und dem Rohrstutzen
- A: Einsetzen der Bohrkrone in den ausreichend geöffneten Durchlass des Rohrstutzens
- B: Vordringen der Bohrkrone durch den Rohstutzen bis zur Wandung bzw. Durchbohren der Wandung
- C: Herausziehen der Bohrkrone aus dem Rohrstutzen
- D: Abdichtendes Positionieren des Modulschafts des Sensormoduls im Durchlass des Rohrstutzens
- E: Verbringen des Sensormoduls in seine endgültige Bestimmungsposition

## Patentansprüche

1. Verfahren zur Montage mindestens eines Sensormoduls (28) oder Sensors (30) in oder an einer durch Beton oder Stahlbeton gebildeten Wandung (6) eines zumindest teilweise mit gärfähigem Material (8) oder Substrat (8) beladenen oder beladbaren Reaktorbehälters (4), mit folgenden Verfahrensschritten:
- Montage eines Flansches (10) mit angesetztem Rohrstutzen (16) an einer Außenseite (6') der Wandung (6) des Reaktorbehälters (4) an der vorgesehenen Einbauposition des Sensors (30),
- Öffnen einer am Rohrstutzen (16) angeordneten und einen Durchlass (22) des Rohrstutzens (16) variabel verschließenden oder öffnenden und/oder freigebenden Verschlusseinrichtung (20),
- Einsetzen einer Bohrkrone (24) in den Rohrstutzen (16) bei geöffnetem Durchlass (22) und Durchbohren der Wandung (6) bis zur Herstellung einer in einen Innenraum (4') des Reaktorbehälters (4) reichenden Durchgangsbohrung (26),
- Herausziehen der Bohrkrone (24) aus dem Rohrstutzen (16) und unmittelbar darauf folgendes Verschließen der Verschlusseinrichtung (20),
- Öffnen der Verschlusseinrichtung (20) und unmittelbar darauf folgendes Montieren eines Sensormoduls (28), dessen Modulschaft (32) weitgehend dichtend im Durchlass (22) des Rohrstutzens (16) positioniert wird, wobei mindestens ein mit einem zur Erfassung wenigstens eines Parameters des im Reaktorbehälter (4) befindlichen Gärsubstrats (8) vorgesehener und/oder ausgebildeter Sensor (30) an einem in den Innenraum (4') des Reaktorbehälters (4) ragenden Abschnitt des Modulschafts (32) angeordnet ist.

2. Verfahren nach Anspruch 1, bei dem der Flansch (10) in einem Bereich der Wandung (6) montiert wird, die bei mit Gärsubstrat (8) beladenem Reaktorbehälter (4) unterhalb einer Gasphase (14') und/oder unterhalb eines Flüssigkeitsspiegels (8') angeordnet ist.

3. Verfahren nach Anspruch 1 oder 2, bei dem ein nach außen aus dem Rohrstutzen (16) reichender Abschnitt des Modulschafts (32) mit elektrischen Anschlüssen (34) und/oder Leitungen (36) zur Übermittlung von elektrischen Sensorsignalen (38) des mindestens einen Sensors (30) des Sensormoduls (28) ausgestattet ist.

4. Verfahren nach einem der Ansprüche 1 bis 3, bei dem der mindestens eine Sensor (30) die Zustandsparameter des im Erfassungsbereich (S) des Sensormoduls (28) befindlichen Innenraumes (4') des Reaktorbehälters (4), insbesondere seiner Materialfüllung (8) und/oder der darin befindlichen Flüssigkeit in elektrische Signale (38) wandelt, die mittels Datenverarbeitungseinrichtungen (40) ausgewertet und/oder verarbeitet werden.

5. Verfahren nach einem der Ansprüche 1 bis 4, bei dem eine an der Wandung (6) anliegende Flanschfläche (10') des Flansches (10) eine mit einer Außenkontur und/oder Wandkrümmung der Wandung (6) des Reaktorbehälters (4) korrespondierende Kontur aufweist.

6. Verfahren nach einem der Ansprüche 1 bis 5, bei dem der Rohrstutzen (16) bei montiertem Flansch (10) ungefähr rechtwinkelig zu einer Oberfläche der Wandung (6) des Reaktorbehälters (4) orientiert ist.

7. Verfahren nach einem der Ansprüche 1 bis 6, bei dem der mindestens eine Sensor (30) des Sensormoduls (28) durch einen Kraftsensor gebildet ist oder einen solchen Kraftsensor umfasst.

8. Verfahren nach Anspruch 7, bei dem der mindestens eine Sensor (30) des Sensormoduls (28) durch eine Wägezelle gebildet ist oder eine solche Wägezelle umfasst.

9. Verfahren nach einem der Ansprüche 1 bis 8, bei dem der mindestens eine Sensor (30) des Sensormoduls (28) durch einen Temperatursensor gebildet ist oder einen solchen Temperatursensor umfasst.

10. Verfahren nach einem der Ansprüche 1 bis 9, bei dem der mindestens eine Sensor (30) des Sensormoduls (28) durch einen Strömungssensor gebildet ist oder einen solchen Strömungssensor umfasst.

11. Verfahren nach einem der Ansprüche 1 bis 10, bei dem der mindestens eine Sensor (30) des Sensormoduls (28) durch eine optische Erfassungseinrichtung gebildet ist oder eine solche optische Erfassungseinrichtung umfasst.

12. Reaktorbehälter (4), der eine durch Beton oder Stahlbeton gebildete Wandung (6) aufweist, und der zumindest teilweise mit gärfähigem Material (8) oder Substrat (8) beladen oder beladbar ist, und der mindestens einen Flansch (10) mit darin montiertem Sensormodul (28) aufweist, wobei der Flansch (10) mit einem Verfahren gemäß einem der Ansprüche 1 bis 11 montiert wurde.

13. Biogasanlage (2) mit mindestens einem Reaktorbehälter (4) gemäß Anspruch 12, der mit wenigstens einem Sensormodul (28) ausgestattet ist, das mit einem Verfahren gemäß einem der Ansprüche 1 bis 11 montiert wurde.
